(19) Europäisches Patentamt
European Patent Office
Office européen des brevets

(11) Publication number: **0 178 580**
**B1**

(12) # EUROPEAN PATENT SPECIFICATION

(45) Date of publication of patent specification: **03.01.90**

(21) Application number: **85112793.6**

(22) Date of filing: **09.10.85**

(51) Int. Cl.[5]: **C 07 D 317/20**, C 07 D 319/06, C 07 C 51/00, C 07 C 57/30

(54) **Intermediates for the synthesis of carboxylic acids.**

(30) Priority: **15.10.84 IT 721384**

(43) Date of publication of application:
**23.04.86 Bulletin 86/17**

(45) Publication of the grant of the patent:
**03.01.90 Bulletin 90/01**

(84) Designated Contracting States:
**AT BE CH DE FR GB IT LI LU NL SE**

(56) References cited:
**EP-A-0 101 124**

**CHEMICAL ABSTRACTS, vol. 67, no. 25, 18th December 1967, pages 10876-10877, abstract no. 115561j, Columbus, Ohio, US; F. ULRICH et al.: "Cardiovascular effects of 1,3-dioxolanes", & ACTA BIOL. MED. GER. 19(1), 129-36 (1967)**

**CHEMICAL ABSTRACTS, vol. 55, no. 23, 13th November 1961, column 23826-g, Columbus, Ohio, US; F. MELSON: "Pharmacological studies on some derivatives of 1,3-dioxolane, with special regard to their action on the central nervous system", & ACTA BIOL. ET MED. GER. 6, 395-406 (1961)**

(73) Proprietor: **ZAMBON S.p.A.**
**Via Cappuccini, 40**
**I-36100 Vicenza (IT)**

(72) Inventor: **Giordano, Claudio**
**via Canove Nuove 23**
**Vicenza (IT)**
Inventor: **Perdoncin, Giulio**
**via Ospedale 30**
**I-36100 Vicenza (IT)**
Inventor: **Brambilla, Adriano**
**via de Gasperi**
**I-36045 Lonigo (Ve) (IT)**
Inventor: **Castaldi, Graziano**
**via Livia Gallina 5**
**Briona (Novara) (IT)**

(74) Representative: **Gervasi, Gemma, Dr. et al**
**Studio Brevetti e Marchi NOTARBARTOLO & GERVASI 33, Viale Bianca Maria**
**I-20122 Milano (IT)**

**EP 0 178 580 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to ketals of alkyl-aryl-ketones and their use in the preparation of alpha-arylalkanoic acids.

Alpha-arylalkanoic acids (or their derivatives) possessing pharmaceutical activity or useful as intermediates in the preparation of antiparasitics are known to be produced when ketals of alkyl-aryl-ketones functionalised in the alpha position in the alkyl part are subjected to a rearrangement reaction.

The rearrangement methods include those described in European patent applications 34871 (Blaschim), 35305 (Blaschim), 48136 (Sagami), 64394 (Syntex), 89711 (Blaschim) and 101124 (Zambon), in Italian patent applications 21841 A/82 (Blaschim and CNR), 22760 A/82 (Zambon) and 19438 A/84 (Zambon) and in the publication J. Chem. Soc., Perkin I, 11, 2575 (1982).

The ketals used as the starting substances in the aforesaid patent applications are exclusively the products of the reaction between suitable alkyl-aryl-ketones and aliphatic alcohols or glycols, i.e. alkanediols or alkenediols.

The alpha-functionalisation, which enables the transposition reaction to be carried out, can be effected either at the ketal level or at the ketone level, ie before the ketalisation reaction.

We have now discovered new ketals, the subject matter of the present invention, of formula:

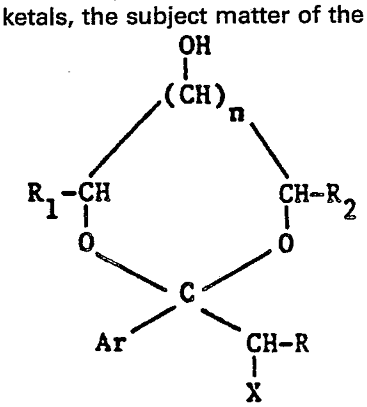

(I)

in which:

Ar represents an aryl, possibly substituted;

R represents a $C_1$—$C_4$ alkyl;

X represents a hydrogen, chlorine, bromine or iodine atom, a hydroxyl, or an acyloxy, alkylsulphonyloxy or arylsulphonyloxy group;

n represents 0 or 1, where if n = 0 one of $R_1$ and $R_2$ represents a —$CH_2OH$ groups and the other represents a hydrogen atom, and if n = 1 both $R_1$ and $R_2$ represent hydrogen atoms.

On rearrangement, the compounds of formula I form the corresponding alpha-arylalkanoic acids or their immediate precursors.

The compounds of formula I are prepared by reacting glycerin with a ketone of formula

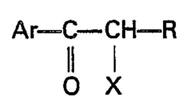

(II)

in which Ar, X and R have the meanings given for formula I.

The ketalisation reaction can be conducted by conventional methods, using azeotropic distillation or chemical agents to remove the water which forms during the course of the reaction.

Alternatively, the reaction between the glycerin and the ketone of formula II can be conducted in the presence of thionyl chloride in accordance with the method described in the copending Italian patent application 7205 A/84 in the name of the present applicant.

The compounds of formula I can also be prepared by reacting the ketone of formula II with other compounds which with regard to the preparation of the compounds of formula I can be defined as glycerin analogues.

These glycerin analogues comprise 2,3-epoxy-propan-1-ol, 2,3-epoxy-1-chloropropane (then transforming the chloromethyl group into hydroxymethyl) and glycerol carbonate (4-hydroxymethyl-2-keto-1,3-dioxolane).

Useful precursors of the compounds of formula I are also the O-(2,3-dihydroxypropyl) derivatives of the enolethers corresponding to the ketones of formula II.

The compounds of formula I can also be prepared by transacetalation, ie by reacting ketals (for example dimethylketals) of the compounds of formula II with glycerin.

The aforesaid reactions are known, but only in relation to the preparation of acetals of aldehydes or of hexafluoroacetone (Synthesis, 501, 1981).

The ketalisation reaction can lead to the formation of 5 to 6-term cyclic ketals of formula I—A and I—B respectively:

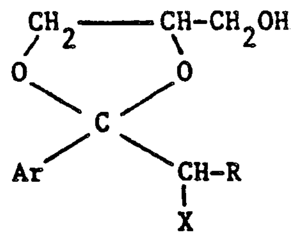

(I-A)

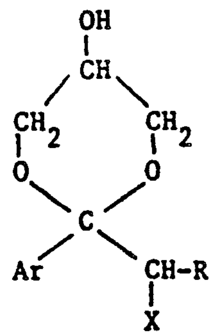

(I-B)

The ketalisation reacton generally leads to the formation of mixtures of ketals of formula I—A and I—B. If required, even though not necessary for the preparation of alpha-arylalkanoic acids, it is possible to prepare the individual compounds of formula I—A or I—B by protecting one of the glycerin hydroxyls before the ketalisation reaction, and deprotecting the hydroxyl when the ketal has been formed.

The compounds of formula I—A comprise two asymmetric carbon atoms in the cyclic structure, and a third asymmetric carbon atom is present when the substituent X is other than hydrogen. They can therefore exist in the form of four pairs of enantiomers.

The compounds of formula I—B can exist in the form of two geometric isomers. Furthermore, when X is other than hydrogen an asymmetric carbon atom is present, and thus each of the two geometric isomers can be present in the form of one pair of enantiomers.

Both the compounds of formula I—A and the compounds of formula I—B and their respective stereoisomers form the corresponding alpha-arylalkanoic acids on rearrangement.

It is therefore unnecessary to separate the ketalisation products for the rearrangement reaction, and they can be used as such even in the form of the mixture.

Thus the present invention covers both the mixtures of compounds I—A and I—B and their respective stereoisomers obtained by synthesis or by a balancing reaction, and the compounds pertaining to a single structure, obtained for example by chromatographic separation.

Likewise, the invention also covers both the mixtures of the various stereoisomers obtained by the synthesis, and the mixtures or the individual isomers obtained by separation, including partial separation.

The compounds of formula I in which X is H or OH are intermediates for the preparation of the other compounds of formula I, which are prepared by halogenation or by acylation in accordance with known methods.

The compounds of formula I in which X is other than H and OH form the corresponding alpha-arylalkanoic acids on rearrangement.

The compounds of formula I in which X is other than H and OH can be rearranged to alpha-arylalkanoic acids by applying the procedures described in the known art. Depending on the reaction conditions, either the acids are obtained directly or their immediate precursors such as esters which on hydrolysis form the required acids.

The ketals of formula I in which X is a halogen atom are of more immediate preparation, by the ketalisation of an alpha-haloketone of formula II (in which X is halogen) or by the halogenation of a ketal of formula I in which X is H.

Exclusively for this reason, the compounds of formula I in which X represents a hydrogen, chlorine, bromine or iodine atom represent a preferred species of the invention.

These ketals can be rearranged in accordance with the methods described in the literature.

It is however preferable to conduct the rearrangement reaction in a protic polar medium (which can be the glycerin itself) in a neutral or slightly alkaline environment, possibly in the presence of an inert diluent as described in Italian patent application 22760 A/82 (Zambon) and in European patent application 101124 (Zambon).

The preference for this rearrangement method is due to its practical advantages in terms of its ease of execution and of the fact that it does not require the presence of metal salts as catalysts.

The compounds of formula I also possess an important property not shared by the known ketals used as intermediates in the preparation of alpha-arylalkanoic acids.

This property is that they are able to form alpha-arylalkanoic acids or their immediate precursors by an

autosolvolytic rearrangement reaction.

This means that by heating a ketal of formula I in which X is other than H and OH, either in the absence or in the presence of an inert diluent, the corresponding alpha-arylalkanoic acid (or one of its immediate precursors) is directly obtained.

The main alpha-arylalkanoic acids known for their pharmaceutical properties or as intermediates in the synthesis of antiparasitics include (2-(4-isobutylphenyl)-propionic acid known as Ibuprofen, 2-(3-phenoxyphenyl)-propionic acid known as Fenopren, 2-(2-fluoro-4-diphenylyl)-propionic acid known as Flurbiprofen, 2-[4-(2-thienyl-carbonyl)-phenyl]-propionic acid known as Suprofen, 6-methoxy-2-naphthyl-propionic acid of which the S(+) isomer is known as Naproxen and its 5-bromo-6-methoxy-2-naphthyl-propionic and 5-chloro-6-methoxy-2-naphthyl-propionic precursors, and 2-(4-chlorophenyl)-3-methyl-butyric acid and 2-(4-difluoromethoxyphenyl)-3-methylbutyric acid, these latter two being useful as intermediates in the preparation of antiparasitics.

Consequently specific meanings of the substituent Ar comprise 6-methoxy-2-naphthyl, 5-bromo-6-methoxy-2-naphthyl, 5-chloro-6-methoxy-2-naphthyl, 4-isobutyl-phenyl, 4-chlorophenyl, 4-difluoro-methoxy-phenyl, 3-phenoxy-phenyl, 4-(2-thienylcarbonyl)-phenyl and 2-fluoro-4-diphenyl, and the specific meanings of R are methyl and isopropyl.

The present invention is illustrated in detail hereinafter by means of the following examples.

## Example 1

Preparation of 2-(4-isobutylphenyl)-propionic acid (Ibuprofen)

Glycerin (22.4 g; 0.24 moles) is added to a solution of 2-chloro-4'-isobutyl-propiophenone (22.4 g; 0.1 moles) in isopropanol (22.4 g) kept under agitation in a nitrogen atmosphere at 20°C ± 2°C.

The solution is heated to 50°C and thionyl chloride (15.5 g; 0.13 moles) is added over 30 minutes while keeping the temperature constant.

The system is kept under agitation in a nitrogen atmosphere at 50°C ± 2°C for three hours.

The solution is then concentrated under vacuum and the oily residue is added over 30 minutes to a suspension of sodium bicarbonate (15 g; 0.178 moles) in water (150 ml) while maintaining the temperature at 20°C ± 2°C.

The lower layer is separated and the aqueous layer is extracted with methylene chloride (50 g).

The organic phases are pooled and washed with a solution of sodium bicarbonate (1.5 g; 0.017 moles) in water (100 g), the solvent then being removed by evaporation under reduced pressure.

33 g of an oil are obtained consisting of a mixture of the isomers 2-(4-isobutylphenyl)-2-(1-chloroethyl)-4-hydroxymethyl-1,3-dioxolane and 2-(4-isobutylphenyl)-2-(1-chloroethyl)-5-hydroxy-1,3-dioxane.

Glycerin (30 g) and potassium acetate (3.4 g; 0.0346 moles) are added to a portion of the residue (10 g); the solution is heated under agitation and under nitrogen to an internal temperature of 185°C, this temperature being maintained for 17 hours.

The solution is then cooled to ambient temperature, diluted with dimineralised water (10 g) and extracted with 1,2-dichloroethane (3 × 30 g).

The organic extracts are concentrated under vacuum and the residue is taken up in 10.4 g of a 30% solution of sodium hydroxide in water (150 g).

After two hours under agitation at ambient temperature, the basic solution is extracted with dichloroethane (3 × 5 g).

The basic aqueous phase is acidified to pH 1.5 with concentrated hydrochloric acid, to precipitate the 4-isobutylphenylpropionic acid which is washed and dried under vacuum at 40°C (4.7 g; 0.022 moles).

## Example 2

Preparation of 2-(6-methoxy-2-naphthyl)-propionic acid

A) Glycerin (100 g; 1.08 moles) is added to a solution of 2-bromo-1-(6-methoxy-2-naphthyl)-propan-1-one (100 g; 0.34 moles) in isopropanol (100 g) kept under agitation in a nitrogen atmosphere at 20°C ± 2°C.

The solution is heated to 50°C and thionyl chloride (52.6 g; 0.44 moles) is added over 30 minutes, while maintaining constant temperature.

The system is kept under agitation and under nitrogen at 50°C ± 2°C for five hours.

The solution is then concentrated under vacuum and the oily residue is added over 30 minutes to a suspension of sodium bicarbonate (102 g; 1.21 moles) in water (680 g) while maintaining the temperature at 20°C ± 2°C.

The lower layer is separated and the aqueous layer is extracted with methylene chloride (100 g).

The organic phases are pooled and washed with a solution of sodium bicarbonate (10.2 g; 0.12 moles) in water (340 g). The solvent is then removed by evaporation under reduced pressure.

121.3 g of residue are obtained consisting of a mixture of the isomers 2-(6-methoxy-2-naphthyl)-2-(1-bromoethyl)-4-hydroxymethyl-1,3-dioxolane, 2-(6-methoxy-2-naphthyl)-2-(1-chloroethyl)-4-hydroxymethyl-1,3-dioxolane, 2-(6-methoxy-2-naphthyl)-2-(1-bromoethyl)-5-hydroxy-1,3-dioxane, and 2-(6-methoxy-2-naphthyl)-2-(1-chloroethyl)-5-hydroxy-1,3-dioxane.

B) Ethylene glycol (10.6 g) and potassium acetate (1.5 g; 0.015 moles) are added to a portion of the residue (5 g).

The suspension is heated under agitation in a nitrogen atmosphere to an internal temperature of 150°C

and is maintained at this temperature for 20 hours.

After cooling to ambient temperature, the solution is diluted with water (20 g), acidified to pH 4.5 and extracted three times with dichloroethane (3 × 50 g).

The extracts are concentrated under vacuum, and 3.6 g of a 30% solution of sodium hydroxide in dimineralised water (50 g) are added to the oily residue.

The mixture is heated to boiling for two hours, the solution is then cooled to ambient temperature and extracted with dichloroethane (100 g).

The aqueous phase is acidified to pH 1.5 with 30% hydrochloric acid to precipitate the 2-(6-methoxy-2-naphthyl)-propionic acid which is filtered off, washed with water and dried under vacuum at 40°C to give 2.4 g (yield 74.5%), M.P. 154°C.

Example 3

Glycerin (10.6 g) and potassium acetate (1.5 g; 0.015 moles) are added to a 5 g portion of the mixture of 1-(6-methoxy-2-naphthyl)-propan-1-one ketals obtained as described in Example 2 paragraph A.

The suspension is heated under agitation in a nitrogen atmosphere to an internal temperature of 150°C and is maintained at this temperature for 20 hours.

After cooling to ambient temperature, the solution is diluted with water (20 g), acidified to pH 4.5 and extracted three times with dichloroethane (3 × 50 g).

The extracts are concentrated under vacuum, and 3.6 g of a 30% solution of sodium hydroxide in water (50 g) are added to the oily residue.

The mixture is heated to boiling for two hours, the solution is then cooled to ambient temperature and extracted with dichloroethane (100 g).

The aqueous phase is acidified to pH 1.5 with 37% hydrochloric acid the precipitate the 2-(6-methoxy-2-naphthyl)-propionic acid which is filtered off, washed with water and dried under vacuum at 40°C, to obtain 2.4 g (yield 74.5%), M.P. 153°C.

Example 4

Potassium acetate (5.2 g; 0.052 moles) is added to a 17 g portion of the mixture of 1-(6-methoxy-2-naphthyl)-propan-1-one ketals obtained as described in Example 2, paragraph A; the suspension is heated under agitation in a nitrogen atmosphere to an internal temperature of 170°C and maintained at this temperature for 19 hours.

After cooling to 90°C, the suspension is diluted with 150 ml of water, further cooled to ambient temperature, acidified to pH 4.5 with 37% HCl, and extracted three times with dichloroethane (3 × 70 g).

The extracts are concentrated under vacuum, and water (150 g) and a 30% sodium hydroxide solution (43 g) are added to the oily residue.

The solution is heated under reflux for two hours and then cooled to 20°C and extracted with dichloroethane (200 g), and then further washed with dichloroethane (50 g).

The aqueous phase is concentrated under vacuum to remove the residual solvent, and is then acidified to pH 1.5 with concentrated HCl.

2-(6-methoxy-2-naphthyl)-propionic acid precipitates and is filtered off, washed with water and dried under vacuum at 40°C, to obtain 5.7 g (yield 52%), M.P. 153°C.

**Claims for the Contracting States: BE CH DE FR GB IT LI LU NL SE**

1. Compounds of formula

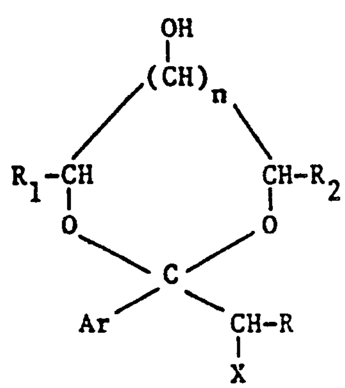

(I)

in which

Ar represents 6-methoxy-2-naphthyl, 5-bromo-6-methoxy-2-naphthyl, 5-chloro-6-methoxy-2-naphthyl, 4-isobutyl-phenyl, 4-chloro-phenyl, 4-difluoro-methoxy-phenyl, 3-phenoxy-phenyl, 4-(2-thienyl-carbonyl)-phenyl and 2-fluoro-4-diphenyl;

R represents a $C_1$—$C_4$ alkyl;

X represents chlorine, bromine or iodine atom, a hydroxyl, or an acyloxy, alkylsulphonyloxy or arylsulphonyloxy group;

n represents 0 or 1, where if n = 0 one of $R_1$ and $R_2$ represents a $CH_2$—OH group and the other represents a hydrogen atom, and if n = 1 both $R_1$ and $R_2$ represent hydrogen atoms.

2. Compounds as claimed in claim 1 of formula:

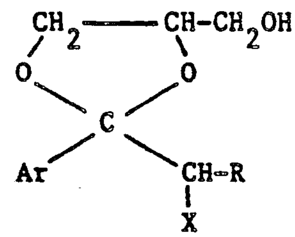

(I-A)

in which Ar, R and X are as above defined.

3. Compounds as claimed in claim 1 of formula:

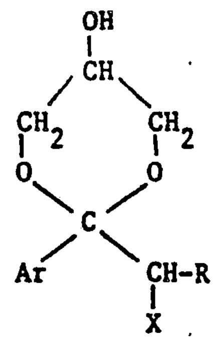

(I-B)

in which Ar, R and X are as above defined.

4. Compounds as claimed in claim 1, wherein X represents a chlorine, bromine or iodine atom.

5. Compounds as claimed in claim 1, wherein Ar represents a group chosen from 6-methoxy-2-naphthyl, 5-bromo- or 5-chloro-6-methoxy-2-naphthyl and R represents a methyl.

6. Compounds as claimed in claim 1 wherein Ar represents a 4-iso-butyl-phenyl and R represents a methyl.

7. A process for preparing the compounds of claim 1, wherein a ketone of formula

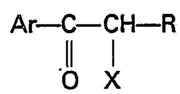

(II)

in which Ar, X and R are as above defined, is subjected to ketalisation reaction with glycerin or one of its analogues.

8. A process as claimed in claim 8 wherein a ketone of formula (II) is reacted with a glycerin analogue chosen from 2,3-epoxy-propan-1-ol, 2,3-epoxy-1-chloropropane and 4-hydroxymethyl-2-keto-1,3-dioxolane.

9. A process for preparing alpha-arylalkanoic acids wherein a ketal according to claim 1, in which X is other than OH, is subjected to a rearrangement reaction.

10. A process for preparing alpha-arylalkanoic acids as claimed in claim 9, characterised in that the rearrangement reaction is conducted in a protic polar medium in a neutral or weakly alkaline environment, possibly in the presence of an inert diluent.

11. A process for preparing alpha-arylalkanoic acids as claimed in claim 9, characterised in that the rearrangement reaction is effected by simply heating the ketal, possibly in the presence of an inert diluent.

**Claims for the Contracting State: AT**

1. A process for preparing compounds of formula

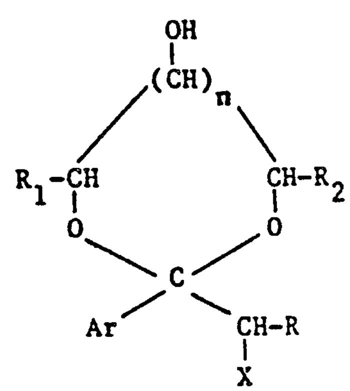

(I)

in which

Ar represents 6-methoxy-2-naphthyl, 5-bromo-6-methoxy-2-naphthyl, 5-chloro-6-methoxy-2-naphthyl, 4-isobutyl-phenyl, 4-chloro-phenyl, 4-difluoro-methoxy-phenyl, 3-phenoxy-phenyl, 4-(2-thienyl-carbonyl)-phenyl and 2-fluoro-4-diphenyl;

R represents a $C_1$—$C_4$ alkyl;

X represents chlorine, bromine or iodine atom, a hydroxyl, or an acyloxy, alkylsulphonyloxy or arylsulphonyloxy group;

n represents 0 or 1, where if n = 0 one of $R_1$ and $R_2$ represents a $CH_2$—OH group and the other represents a hydrogen atom, and if n = 1 both $R_1$ and $R_2$ represent hydrogen atoms wherein a ketone of formula

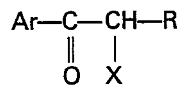

(II)

in which Ar, X and R are as above defined, is subjected to ketalisation reaction with glycerin or one of its analogues.

2. A process as claimed in claim 1 wherein a ketone of formula (II) is reacted with a glycerin analogue chosen from 2,3-epoxy-propan-1-ol, 2,3-epoxy-1-chloropropane and 4-hydroxymethyl-2-keto-1,3-dioxolane.

3. A process for preparing alpha-arylalkanoic acids, wherein a ketal of formula (1), in which X is other than OH, is subjected to a rearrangement reaction.

4. A process for preparing alpha-arylalkanoic acids as claimed in claim 3, characterised in that the rearrangement reaction is conducted in a protic polar medium in a neutral or weakly alkaline environment, possibly in the presence of an inert diluent.

5. A process for preparing alpha-arylalkanoic acids as claimed in claim 3, characterised in that the rearrangement reaction is effected by simply heating the ketal, possibly in the presence of an inert diluent.

**Patentansprüche für die Vertragsstaaten: BE CH DE FR GB IT LI LU NL SE**

1. Verbindungen der Formel

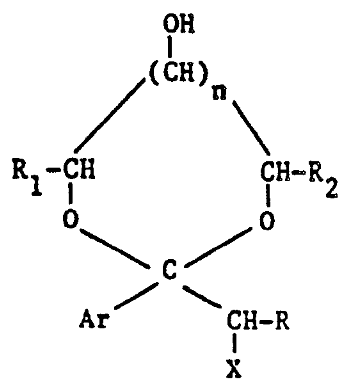

(I)

worin Ar bedeutet: 6-Methoxy-2-naphthyl, 5-Brom-6-methoxy-2-naphthyl, 5-Chlor-6-methoxy-2-naphthyl, 4-Isobutylphenyl, 4-Chlorphenyl, 4-Difluormethoxyphenyl, 3-Phenoxyphenyl, 4-(2-thienylcarbonyl)phenyl und 2-Fluor-4-diphenyl; R bedeutet ein $C_1$—$C_4$-Alkyl, X bedeutet: Chlor, Brom oder Jod, Hydroxyl oder Acyloxy, Alkylsulfonyloxy oder Arylsulfonyloxy; n bedeutet 0 oder 1, wobei, wenn n = 0, einer von $R_1$ und $R_2$ eine $CH_2OH$-Gruppe und der andere ein Wasserstoffatom bedeuten, und, wenn n = 1 ist, sowohl $R_1$ als auch $R_2$ Wasserstoff bedeuten.

2. Verbindungen nach Anspruch 1 der formel

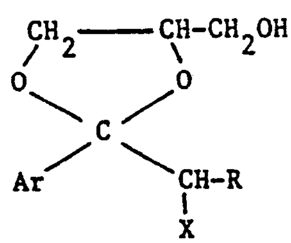

(I-A)

worin Ar, R und X die obige Bedeutung haben.

3. Verbindungen nach Anspruch 1 der Formel

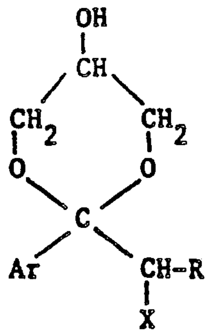

(I-B)

worin Ar, R und X die obige Bedeutung haben.

4. Verbindungen nach Anspruch 1, worin X Chlor, Brom oder Jod bedeutet.

5. Verbindungen nach Anspruch 1, worin Ar eine Gruppe, ausgewählt aus 6-Methoxy-2-naphthyl, 5-Brom- oder 5-Chlor-6-methoxy-2-naphthyl bedeuten und R Methyl ist.

6. Verbindungen nach Anspruch 1, worin Ar 4-Isobutylphenyl und R Methyl bedeutet.

7. Verfahren zur Herstellung der Verbindungen nach Anspruch 1, worin ein Keton der Formel

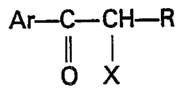

(II),

worin Ar, X und R die obige Bedeutung haben, mit Glyzerin oder einem seiner Analoga der Ketalisierungsreaktion unterworfen wird.

8. Verfahren nach Anspruch 1, worin ein Keton der Formel (II) mit einem Glyzerinanalogon, ausgewählt aus 2,3-Epoxy-propan-1-ol, 2,3-Epoxy-1-chlorpropan und 4-Hydroxymethyl-2-keto-1,3-dioxolan umgesetzt wird.

9. Verfahren zur Herstellung von α-Arylalkansäuren, worin ein Ketal gamäß Anspruch 1, worin X von OH verschieden ist, einer Umlagerungsreaktion unterworfen wird.

10. Verfahren zur Herstellung von α-Arylalkansäuren nach Anspruch 9, dadurch gekennzeichnet, daß die Umlagerungsreaktion in einem protischen polaren Medium in neutraler oder schwach alkalischer Umgebung, gegebenenfalls in Anwesenheit eines inerten Verdünnungsmittels, durchgeführt wird.

11. Verfahren zur Herstellung von α-Arylalkansäuren gemäß Anspruch 9, dadurch gekennzeichnet, daß die Umlagerungsreaktion durch einfaches Erhitzen des Ketals, gegebenenfalls in Anwesenheit eines inerten Verdünnungsmittels, durchgeführt wird.

**Patentansprüche für den Vertragsstaat: AT**

1. Verfahren zur Herstellung von Verbindungen der Formel

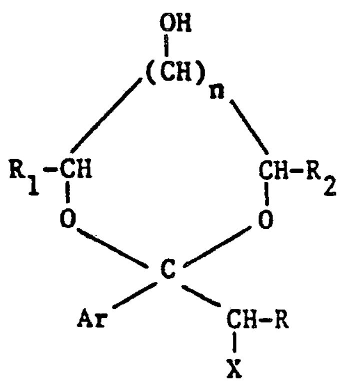

(I)

worin Ar bedeutet: 6-Methoxy-2-naphthyl, 5-Brom-6-methoxy-2-naphthyl, 5-Chlor-6-methoxy-2-naphthyl, 4-Isobutylphenyl, 4-Chlorphenyl, 4-Difluormethoxyphenyl, 3-Phenoxyphenyl, 4-(2-thienylcarbonyl)phenyl und 2-Fluor-4-diphenyl; R bedeutet ein $C_1$—$C_4$-Alkyl, X bedeutet: Chlor, Brom oder Jod, Hydroxyl oder Acyloxy, Alkylsulfonyloxy oder Arylsulfonyloxy; n bedeutet 0 oder 1, wobei, wenn n = 0, einer von $R_1$ und $R_2$ eine $CH_2OH$-Gruppe und der andere ein Wasserstoffatom bedeuten, und, wenn n = 1 ist, sowohl $R_1$ als auch $R_2$ Wasserstoff bedeuten, worin ein Keton der Formel

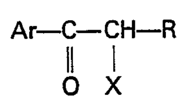

(II),

worin Ar, X und R die obige Bedeutung haben, mit Glyzerin oder einem seiner Analoga der Ketalisierungsreaktion unterworfen wird.

2. Verfahren nach Anspruch 1, worin eine Keton der Formel (II) mit einem Glyzerinanalogon, ausgewählt aus 2,3-Epoxypropan-1-ol, 2,3-Epoxy-1-chlorpropan und 4-Hydroxymethyl-2-keto-1,3-dioxolan umgesetzt wird.

3. Verfahren zur Herstellung von α-Arylalkansäuren, worin ein Ketal gamäß Anspruch 1, worin X von OH verschieden ist, einer Umlagerungsreaktion unterworfen wird.

4. Verfahren zur Herstellung von α-Arylalkansäuren nach Anspruch 3, dadurch gekennzeichnet, daß die Umlagerungsreaktion in einem protischen polaren Medium in neutraler oder schwach alkalischer Umgebung, gegebenenfalls in Anwesenheit eines inerten Verdünnungsmittels, durchgeführt wird.

5. Verfahren zur Herstellung von α-Arylalkansäuren gemäß Anspruch 3, dadurch gekennzeichnet, daß die Umlagerungsreaktion durch einfaches Erhitzen des Ketals, gegebenenfalls in Anwesenheit eines inerten Verdünnungsmittels, durchgeführt wird.

**Revendications pour les Etats contractants: BE CH DE FR GB IT LI LU NL SE**

1. Composés de formule:

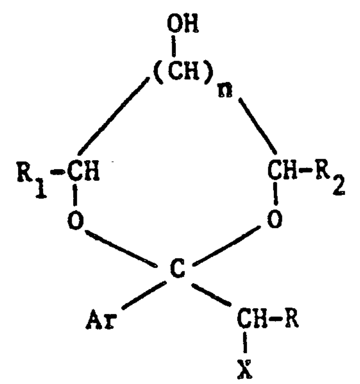

(I)

dans laquelle Ar représente le 6-méthoxy-2-naphtyle, le 5-bromo-6-méthoxy-2-naphtyle, le 5-chloro-6-méthoxy-2-napthyle, le 4-isobutyl-phényle, le 4-chloro-phényle, le 4-difluoro-méthoxy-phényle, le 3-phénoxy-phényle, le 4-(2-thiényl-carbonyl)-phényle et le 2-fluoro-4-diphényle; R représente un $C_1$—$C_4$ alkyle; X représente un atome de chlore, de brome, ou d'iode, un groupe hydroxyle ou acyloxy, alkylsulfonyloxy ou arylsulfonyloxy; n représente 0 ou 1, où si n = 0 l'un des $R_1$ et $R_2$ représente un groupe $CH_2$—OH et l'autre représente un atome d'hydrogène et si n = 1, $R_1$ et $R_2$ représentent chacun un atome d'hydrogène.

2. Composes selon la revendication 1, de formule:

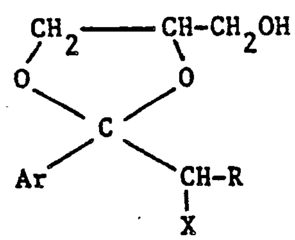

(I-A)

dans laquelle Ar, R et X ont la signification donnée ci-dessus.

3. Composés selon la revendication 1, de formule:

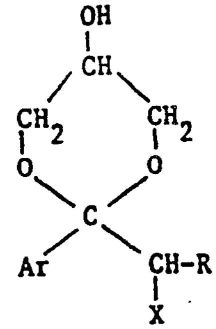

(I-B)

dans laquelle Ar, R et X ont la signification donnée ci-dessus.

4. Composés selon la revendication 1, dans laquelle X représente un atome de chlore, de brome ou d'iode.

5. Composés selon la revendication 1, dans laquelle Ar représente un groupe choisi parmi le 6-méthoxy-2-naphtyle, le 5-bromo ou 5-chloro-6-méthoxy-2-napthyle et R représente la radical méthyle.

6. Composés selon la revendication 1, dans laquelle Ar représente le groupement 4-iso-butyl-phényle et R représente le radical méthyle.

7. Procédé de préparation des composés de la revendication 1, dans lequel une cétone de formule

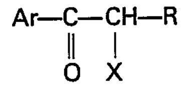 (II),

(où Ar, X et R ont la signification donnée ci-dessus) est soumise à une réaction de cétalisation avec la glycérine ou un composé analogue.

8. Procédé selon la revendication 7, caractérisé en ce qu'on fait réagir une cétone de formule (II) avec un composé analogue à la glycérine choisi parmi le 2-3 époxy-propan-1-ol, la 2,3-époxy-1-chloropropane et la 4-hydroxyméthyl-2-céto-1,3-dioxolane.

9. Procédé de préparation des acides α-arylalcanoïques dans lequel on soumet un cétal selon la revendication 1, dans lequel X est autre que OH, à une réaction de réarrangement.

10. Procédé de préparation des acides α-arylalcanoïques selon la revendication 9, caractérisé en ce que la réaction de réarrangement est effectuée dans un milieu polaire protique en environnement neutre ou faiblement alcalin, si possible en présence d'un diluant inerte.

11. Procédé de préparation des acides α-arylalcanoïques selon la revendication 9, caractérisé en ce que la réaction de réarrangement est effectuée par simple chauffage du Cétal, si possible en présence d'un diluant inerte.

**Revendications pour l'Etat contractant: AT**

1. Procédé de préparation de composés de formule:

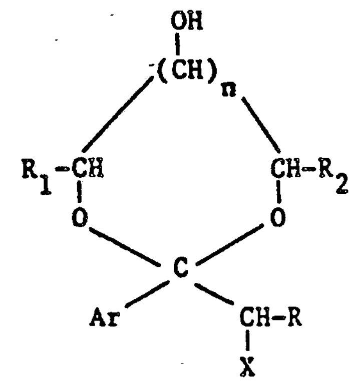 (I)

dans laquelle Ar représente le 6-méthoxy-2-naphtyle, le 5-bromo-6-méthoxy-2-naphtyle, le 5-chloro-6-méthoxy-2-napthyle, le 4-isobutyl-phényle, le 4-chloro-phényle, le 4-difluoro-méthoxy-phényle, le 3-phénoxy-phényle, le 4-(2-thiényl-carbonyl)-phényle et le 2-fluoro-4-diphényle; R représente un $C_1$—$C_4$ alkyle; X représente un atome de chlore, de brome, ou d'iode, un groupe hydroxyle ou acyloxy, alkylsulfonyloxy ou arylsulfonyloxy; n représente 0 ou 1, où si n = 0 l'un des $R_1$ et $R_2$ représente un groupe $CH_2$—OH et l'autre représente un atome d'hydrogène et si n = 1, $R_1$ et $R_2$ représentent chacun un atome d'hydrogène.

Suivant lequel une cétone de formule:

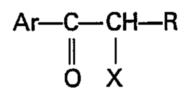 (II),

(où Ar, X et R ont la signification donnée ci-dessus) est soumise à une réaction de cétalisation avec la glycérine ou un composé analogue.

2. Procédé selon la revendication 1, caractérisé en ce qu'on fait réagir une cétone de formule (II) avec un composé analogue à la glycérine choisi parmi le 2-3 époxy-propan-1-ol, la 2,3-époxy-1-chloropropane et la 4-hydroxyméthyl-2-céto-1,3-dioxolane.

3. Procédé de préparation des acides α-arylalcanoïques dans lequel on soumet un cétal de formule (I), dans lequel X est autre que OH, à une réaction de réarrangement.

4. Procédé de préparation des acides α-arylalcanoïques selon la revendication 3, caractérisé en ce que la réaction de réarrangement est effectuée dans un milieu polaire protique en environnement neutre ou faiblement alcalin, si possible en présence d'un diluant inerte.

5. Procédé de préparation des acides α-arylalcanoïques selon la revendication 3, caractérisé en ce que la réaction de réarrangement est effectuée par simple chauffage du Cétal, si possible en présence d'un diluant inerte.